# EUROPÄISCHE PATENTSCHRIFT

(11) **EP 2 224 970 B1**
(45) Veröffentlichungstag und Bekanntmachung des Hinweises auf die Patenterteilung: **28.02.2018**
(21) Anmeldenummer: 08848649.3
(22) Anmeldetag: 12.11.2008
(51) Int. Cl.: A61L 27/32, A61L 27/54, A61L 27/42

(54) **IMPLANTAT UND VERFAHREN ZUR BESCHICHTUNG EINES IMPLANTATS**
IMPLANT AND METHOD FOR COATING AN IMPLANT
IMPLANT ET PROCÉDÉ DE REVÊTEMENT D'UN IMPLANT

(30) Priorität: 12.11.2007 DE 102007054214
(43) Veröffentlichungstag der Anmeldung: 08.09.2010
(73) Patentinhaber: Medicoat AG, 5506 Mägenwil (CH)
(72) Erfinder: GRUNER, Heiko, CH-5712 Beinwil am See (CH); GRUNER, Philipp, CH-5612 Villmergen (CH); TOURENNE, Francis, 90100 Faverois (FR)
(74) Vertreter: Otten, Roth, Dobler & Partner mbB Patentanwälte
(86) Internationale Anmeldenummer: PCT/EP2008/009519
(87) Internationale Veröffentlichungsnummer: WO 2009/062671

(56) Entgegenhaltungen:
- WO-A2-2007/022211
- SUN L ET AL: "Material fundamentals and clinical performance of plasma-sprayed hydroxyapatite coatings: A review" JOURNAL OF BIOMEDICAL MATERIALS RESEARCH 2001 US, Bd. 58, Nr. 5, 2001, Seiten 570-592, XP002569434 ISSN: 0021-9304
- CHEN W ET AL: "In vitro anti-bacterial and biological properties of magnetron co-sputtered silver-containing hydroxyapatite coating" BIOMATERIALS, ELSEVIER SCIENCE PUBLISHERS BV., BARKING, GB, Bd. 27, Nr. 32, 1. November 2006 (2006-11-01), Seiten 5512-5517, XP025097379 ISSN: 0142-9612 [gefunden am 2006-11-01]

## Beschreibung

Seit 1985 werden künstliche Gelenkteile serienmässig mit einer durch verschiedene Verfahren des thermischen Spritzens aufgebrachten Kalziumphosphatschicht für die rasche und zwangsweise Osseointegration im umgebenden Knochengewebe vorbereitet. Diese Technik geht auf eine japanische Patentanmeldung zurück (Sumitomo Chemical Co., Jap. Patentapplication No. 158.745 (1975)) und wurde im Laufe der Jahre immer weiter zur Lösung der genannten Aufgabenstellung optimiert. Aktuell kommt hauptsächlich das Vakuum-Plasmaspritzverfahren (VPS) für die Schichtbildung zur Anwendung. Dieses Verfahren ermöglicht es, die zur Schichtbildung aufgeschmolzenen Kalziumphosphat-Pulverpartikel nur kurz oberhalb der benötigten Schmelztemperatur zu halten, wodurch kritische Phasenumwandlungen beim Wiedererstarren minimalisiert werden, und trotzdem eine mechanisch stabile, fest haftende Schicht keramischer Natur entsteht. Die genaue Schilderung der Prozessvorgänge und Optimierungsschritte sind zum Beispiel detailliert im Beitrag "Thermal Sprayed Coatings on Titanium" im Buch "Titanium in Medicine", ISBN 3-540-66936-1, Springer-Verlag Berlin, beschrieben.

### Stand der Technik

Kalziumphosphatschichten werden in der Prothetik in zwei unterschiedlichen Anwendungsfällen verwendet, welche sich durch die gewünschte Verweilzeit des Implantates im menschlichen Körper unterscheiden.

In der Traumatologie, bei welcher ein Implantat die Ausheilung von Knochenbrüchen zeitlich beschränkt unterstützt, soll die Kalziumphosphatschicht eine definierte Löslichkeit und/oder eine zeitlich begrenzte mechanische Stabilität besitzen. So sorgt sie auf der einen Seite zunächst für eine perfekte mechanische Fixation im Knochen/Schichtverbund, wichtig für das rasche Verwachsen der gebrochenen Knochenteile an den zueinander einjustierten Berührungsflächen. Auf der anderen Seite soll diese Unterstützung durch das langsame Verschwinden der Kalziumphosphatschicht parallel zum Bruchheilprozess kontinuierlich wieder verloren gehen, so dass am Ende der vorgesehenen Verweilzeit diese Osteosynthese-Implantate wieder leicht explantiert werden können.

In der Orthopädie, immer dann wenn ein permanent im Körper verbleibendes Implantat benötigt wird (sogenannte Endoprothesen), soll die Kalziumphosphatschicht so strukturiert sein, dass der einmal gebildete direkte Knochen/Schichtverbund über die gesamte Verweilzeit des Implantates im Körper stabil bleibt und bei jeder Bewegung für eine direkte Lasteinleitung in das Implantat sorgt.

Obwohl durch diesen Kalziumphosphat-Tarnkappenüberzug das künstliche Implantat vom umgebenden Knochen nicht mehr als Fremdkörper erkannt wird, kommt es in 2 - 5 % der Operationen zu einer Abstossreaktion. Das Implantatbett im Knochengewebe entzündet sich. Die Ursache hierfür ist primär in einer bakteriellen Infektion zu suchen, welche entweder unmittelbar nach der Implantation kritische Grösse erreicht, oder sich erst später manifestiert. Die einmal eingefangenen oder schon im Körper vorhandenen Keime werden in der Anfangsphase des Heilprozesses infolge der üblichen Medikamentation still gestellt, um dann nach dem Absetzen der zur Unterstützung der Wundheilung eingesetzten Wirkstoffe oder infolge einer Infektionskrankheit ungehemmt aktiv zu werden. Wenn eine spätere Infektion in der Knochen/Prothesen-Grenzfläche auftritt, helfen Antibiotika-Gaben meist nicht mehr, sie erreichen diesen Ort der Entzündung praktisch nicht. In der Umgebung der Prothese ist das Gewebe vernarbt und schlecht durchblutet. Solche, durch Entzündungs- und Abstossreaktionen erzwungenen Reoperationen bedeuten für die Patienten eine sehr grosse Belastung und verursachen erhebliche Kosten und volkswirtschaftliche Schäden weltweit, mit steigender Tendenz infolge der zunehmenden Verbreitung von gegen Antibiotika multiresistenter Bakterien. Es liegt folglich nahe, nach Lösungen zur wirkungsvollen Vermeidung von frühen oder verzögerten Infektionen und Abstossreaktionen zementlos implantierter Prothesen zu suchen.

Seit langem sind Metalle wie Silber, aber auch Zink oder Kupfer für ihre antibakterielle Wirkung bekannt. Schon in der Antike wurden z.B. Wundsalben mit geschabtem Silber (Ag) versetzt. Silberfäden in die Gazeabdeckung schwerer Brandwunden eingelagert, verhindern effektiv deren bakterielle Entzündung. Durchmesser und Abstand der Ag-Fäden sind so gewählt, dass sie das Wandern und Durchdringen von Bakterien und Viren verhindern. Seit neuestem gibt es auch Ag-beschichtete Textilfasern für diesen Anwendungszweck. Wie bei allen metallischen Wirkstoffen kommt es auch bei Ag auf die richtige Dosierung an, ein Zuviel schädigt den menschlichen Organismus und ein Zuwenig lässt die Dosis wirkungslos verpuffen. Für die antibakterielle Wirksamkeit ist die Konzentration der Metall-Ionen entscheidend. Silber-Ionen (Ag⁺) z.B. können durch Auslösung aus metallischem Silber (Ag⁰) entstehen, oder schon in ionischer Form vorliegen. Hinweise für das richtige Mass der Metallionenzugabe finden sich in der Fachliteratur, auch für den Anwendungsfall der Endoprothesen. Jedoch weichen die Angaben zur Dosierung in der Fachliteratur teilweise stark voneinander ab.

Eine gleichbleibend stabile antibakterielle Wirkung wird z.B. mittels dünnen, durch physikalische oder chemische Abscheidung aus der Dampfphase im Vakuum erzeugte Silberschichten erreicht, aber auch durch galvanisch aufgetragene Silberschichten erzielt. Solche Silberschichten, wie sie z.B. in dem US Patent "Endoprothesis with Galvanised Silver Layer" (US 7,018,411 B2) beschrieben sind, zeigen aber trotz Bioverträglichkeit keine osseokonduktive oder osseoinduktive Wirkung (E. Sheehan et. Al., European Cells and Materials Vol. 10 Suppl. 2, (2001) Seite 75). Trotzdem werden am Klinikum Münster seit 2005 Ag-beschichtete Prothesen mit ausgeprägtem antibakteriellem Erfolg bei Tumorpatienten eingesetzt. Die entzündungsverhindernde Kurzzeitperspektive ist bei diesen Risikopatienten wichtiger als die längerfristige stabile Verankerung.

Auch die antibakterielle Wirkung von Silber-Nanopartikeln wurde untersucht und beschrieben (H.Y. Song et. Al., European Cells and Materials Vol. 11 Suppl. 1, (2006) Seite 58). Die etwa 5 nm grossen Teilchen zeigen ein ausgeprägtes antibakterielles Verhalten und werden als Alternative oder in Ergänzung zu Antibiotika empfohlen. Werden sie zu hochporösen Ag-Partikeln der Grössenordnung 2 bis 10 µm vereint und in der Konzentration von 1 % im Knochenzement für die zementierte Implantationstechnik eingelagert, so entsteht mit Hilfe dieser Ag-Nanoagglomerate eine ausgeprägte antibakterielle Wirkung auch gegen resistente Keime.

Ghani J. et al. berichteten weiter auf dem 53^{rd} Annual Meeting der Orthopaedic Research Society von elektrochemisch abgeschiedenen Hydroxylapatitschichten, welche Ag-Ionen in kontrollierter Weise freisetzen. Die so erzielte antibakterielle Wirkung reicht leider nur für 6 Tage, dann ist die Abgabe der bei der Schichtherstellung eingelagerten Ag-Ionen von der Schichtoberfläche erschöpft. Die Einbringung der Ag-Ionen (Dotierung) erfolgt gleichzeitig mit der elektrochemischen Abscheidung der HA-Schicht. Die Ag-Ionen befinden sich entweder zwischen den HA-Kristalliten oder sind an Stelle von Kalzium Ionen direkt im HA-Kristallgitter eingebaut. Die Freigabe der Ag-Ionen ist folglich nur beim Auflösen der HA-Schicht, oder durch Diffusionseffekte möglich.

Es ist auch bekannt, mittels Sol-Gel Methode und einem zusätzlichen thermischen Temperprozess kristalline HA-Schichten mit unterschiedlichem Gehalt von Ag zu erzeugen, deren Ag-Ionenabgabe zunächst sehr schnell in hoher Konzentration, dann aber nur unter stetiger Abnahme über einen längeren Zeitraum erfolgt (W.C. Chen et al., Key Engineering Materials Vols 330 - 332 (2007), Seite 653 bis 656).

Für antibakterielle Filtermedien wurden poröse HA-Keramiken mit eingelagerten Ag-Ionen entwickelt, wobei der Einbau über einen Ionenaustausch während der Behandlung des porösen Keramikkörpers in einer 0.2 Mol-% AgNO₃-Lösung für etwa 1 Stunde erfolgt.

Interessant in diesem Zusammenhang ist das US-Patent Nr. 5009898 (1989), in dem ein antibakterielles Kalziumphosphatpulver und das Verfahren zu seiner Herstellung beschrieben sind. Neben dem Gehalt von organischen, antibakteriellen Wirkstoffen wie z.B. Protamin wird auch auf die antibakterielle Wirkung eingelagerter Metall-Ionen insbesondere von Silber-Ionen hingewiesen. Gleichzeitig ist eine Vielzahl von Referenzen aufgeführt, welche alle bei der Erteilung des oben aufgeführten Patentes eine Rolle spielten und so den Stand der Technik dokumentieren. Der Metallgehalt im Kalziumphosphat und hier insbesondere in der stofflichen Variante Hydroxylapatit (HA) wird mit sehr grosser Anwendungsbreite von 1 ppm bis 50'000 ppm angegeben. Gemäss beschriebener Methode wird dieser metallionendotierte HA aus einer wässrigen Lösung, in welcher neben Kalzium und Phosphat auch ein Silbersalz gelöst ist, ausgefällt. Entscheidender Patentanspruch ist, dass die Silber-Ionen im Molekül HA gezielt Kalzium-Ionen ersetzen und/oder als einzelne Ionen auf Zwischengitterplätzen des Apatit-Kristallgitters eingebaut sind. Dadurch wird erreicht, dass dieser so modifizierte, antibakterielle HA zusätzlich weitere antibakterielle Substanzen z.B. synthetischer oder organischer Natur durch Absorption aufnehmen kann, ohne dass sich die beiden Wirkstoffe gegenseitig nachteilig beeinflussen. Die Verwendung für dieses antibakterielle HA-Pulver ist in Nahrungsmittel, Kosmetikartikeln, in Zellulose und unter anderem auch im menschlichen Körper (beispielsweise in der Zahnheilkunde) vorgesehen, und zwar immer dann, wenn eine antibakterielle Wirkung benötigt wird. CHEN W ET AL: "In vitro anti-bacterial and biological properties of magnetron co-sputtered silver-containing hydroxyapatite coating",BIOMATERIALS, ELSEVIER SCIENCE PUBLISHERS BV., BARKING, GB, Bd. 27, Nr. 32, 1. November 2006 (2006-11-01), Seiten 5512-5517, offenbart ein zementloses Implantat aus biokompatiblen Materialien, welches einen Grundkörper mit einem sich im Knochen oder Gewebe verankernden Verankerungsbereich aufweist, wobei der Verankerungsbereich zumindest bereichsweise mit einer Deckschicht versehen ist, wobei die Deckschicht unter Anwendung eines Magnetronspritzverfahrens gebildet ist, wobei die Deckschicht im Wesentlichen aus Kalziumphosphat besteht und antibakteriell wirksame Ag-Wirkbestandteile umfasst.

### Aufgabe der Erfindung

Es ist Aufgabe der Erfindung ein Implantat bzw. ein Verfahren zur Beschichtung eines Implantats bzw. eine Deckschicht für ein Implantat vorzuschlagen, welches bzw. welche die vorteilhaften Eigenschaften einer Kalziumphosphat-Schicht, insbesondere einer Hydroxylapatit-Schicht, für das rasche Verwachsen des Knochengewebes mit dem Implantat mit einer signifikanten Verminderung des operativen und postoperativen Infektionsrisikos verbindet, ohne den Prozess des Verwachsens nachteilig zu beeinflussen.

Diese Aufgabe wird ausgehend von den Merkmalen des Oberbegriffs des Anspruchs 1 bzw. 9 bzw. 12 durch die kennzeichnenden Merkmale des Anspruchs 1 bzw. 9 bzw. 12 gelöst. In den jeweiligen Unteransprüchen sind vorteilhafte und zweckmäßige Weiterbildungen angegeben.

Für die Anwendung zementfrei-implantierter Prothesen steht ihre sichere Verankerung im Knochengewebe des Patienten im Vordergrund. Die eingesetzte Beschichtung sollte deshalb uneingeschränkt rasch osseokonduktiv und -induktiv wirken. Dabei soll der intensive Knochen/Prothesenverbund kurzzeitig und/oder längerfristig optimal erhalten bleiben. Ohne diese Eigenschaften zu beeinträchtigen sollen bakterielle Infektionen dank der neuartigen Beschichtung verhindert, oder zumindest stark reduziert werden. Mit Hilfe der erfindungsgemässen Beschichtung erhöht sich folglich die Erfolgswahrscheinlichkeit zementlos implantierten Prothesen, weil das Risiko einer infektiösen Abstossreaktion massgeblich verhindert wird.

### Beschreibung der Erfindung

Die beschriebene Aufgabe wird erfindungsgemäss dadurch gelöst, dass in die, durch thermische Spritztechnik hergestellte Kalziumphosphatschichten, über verschiedene Methoden ein definierter Metallgehalt (insbesondere Silber oder Metalle mit vergleichbarer Wirkung, beispielsweise Zink oder Kupfer, aber auch Mischungen dieser Metalle) eingebaut wird. Unter Kalziumphosphatschichten werden dabei insbesondere Hydroxylapatit (HA), α- und/oder β-Trikalziumphosphat (TCP), Tetrakalziumphosphat (TECP) oder Mischungen dieser Varianten gegebenenfalls mit Zusätzen von Kalziumoxid verstanden. Ohne Beschränkung des Erfindergedankens können auch andere Kalziumphosphate, wie z.B. Pyrophosphat oder wasserfreier Oxyapatit, mit und ohne Zusatz von Kalziumoxid und/oder Fluorapatit verwendet werden.

Eine erste Möglichkeit zur Einstellung des Metallgehaltes nützt das Verfahren eines Ionen-Austausches im Ausgangspulver zur spritztechnischen Herstellung der Implantatbeschichtung. Dabei wird eine festgelegte Anzahl Ca-Ionen im Kristallgitter z.B. durch Ag-Ionen gemäss US-PS 5,009,898 ersetzt und/oder auf Zwischengitterplätzen eingelagert. Eine andere Möglichkeit besteht darin, den Ionen-Austausch erst am schon fertigen Spritzpulver mit der für die thermischen Spritztechnik erforderlichen Kornverteilung durchzuführen. Als dritte Möglichkeit können auch reine, nicht dotierte Kalziumphosphate für die Herstellung des Metall-enthaltenden Spritzpulvers verwendet werden und diese z.B. mit metallischem Silber-Pulver vermischt werden. Die erfindungsgemäss aus diesen aufgeführten Spritzpulvern hergestellten, Ag oder andere Metalle enthaltende Spritzschichten zeichnen sich dadurch aus, dass z.B. das Silber in ionischer Form vorliegt, und/oder in metallischer Form in konkreten Materialportionen fein und gleichmässig in der Schicht verteilt ist.

Auf diese Weise, mittels thermischem Spritzen hergestellte antibakterielle und osseointegrierende Kalziumphosphatschichten, unterscheiden sich sehr charakteristisch von allen anderen bekannten antibakteriellen Schutzschichten.

Zu ihrer erfindungsgemässen, spritztechnischen Herstellung sind zunächst Metall-enthaltende Kalziumphosphat-Spritzpulver (beispielsweise mit Ag) bereitzustellen, jeweils in der Partikelmorphologie, in der Körngrösse und Kornverteilung auf die verschiedenen Methoden des thermischen Spritzens abgestimmt. Für die verschiedenen Spritzverfahren empfehlen sich z.B. dichtgesinterte oder agglomeriert/gesinterte Spritzpulver im Kornbereich auf 10 bis 200 µm, 30 bis 150 µm oder 45 bis 125 µm, bevorzugt aber auch auf 20 bis 60 µm eingestellt oder für besonders feine Schichten und/oder spezielle Spritzverfahren auch auf 5 bis 25 µm.

Werden phasenumwandlungsgefährdete Kalziumphosphate (z.B. HA) ganz oder teilweise für die Schichtherstellung benötigt, so empfehlen sich diejenigen thermischen Spritzmethoden, welche schnelle Prozessgeschwindigkeit bei niedrigeren Partikeltemperaturen und sehr kurzer Verweilzeit der Pulverpartikel oberhalb von Phasenumwandlungstemperaturen ermöglichen (z.B. VPS), um möglichst viel von der Spritzpulverzusammensetzung in die Spritzschicht zu überführen. Im Sinne der Erfindung besteht Pulver bzw. Spritzpulver aus einem Kollektiv einzelner Körner in unterschiedlichen Abmessungen und Formen, die sich wiederum aus feineren Partikeln zusammensetzen aber auch kompakt und in sich homogen sein können.

### Unter Trägerkörnern sind im Sinne der Erfindung

Spritzpulverkörner zu verstehen, welche wenigstens einen Wirkpartikel enthalten oder umfassen. Unter Füllkörnern sind im Sinne der Erfindung Pulverkörner bzw. Spritzpulverkörner zu verstehen, welche keine bzw. keinen Wirkpartikel umfassen.

Die Erfindung wird nun anhand von Zeichnungen und Ausführungsbeispielen näher erläutert.
- Fig. 1a: zeigt eine erste Variante der für die erfindungsgemässe Herstellung benötigten Metall/Kalziumphosphat-Spritzpulverpartikel vor dem Passieren der thermischen Spritzquelle, wobei als antibakterielles Metall Ag gewählt ist.
- Fig. 1b: zeigt das Ag/Kalziumphosphat-Spritzpulverkorn nach dem Passieren der thermischen Spritzquelle und nach dem Aufprall auf der Implantatoberfläche.
- Fig. 2: zeigt schematisch im Schnittbild Details der Schichtstruktur für verschiedene Beispiele erfindungsgemäss hergestellter, antibakterieller, bioaktiver Prothesenbeschichtungen.
- Fig. 3: zeigt eine Variante der Erfindung im schematischen Schnittbild, bevorzugt für die Orthopädie optimiert.
- Fig. 4: zeigt eine erfindungsgemässe Metall/Kalziumphosphat-Spritzschicht im schematischen Schnittbild, wie sie bevorzugt in der Traumatologie angewendet wird.
- Fig. 5: zeigt ein erfindungsgemäßes Implantat und Details der Deckschicht dieses Implantats.
- Fig. 6: zeigt eine schematische Darstellung eines thermischen Spritzsystems.
- Fig. 7: zeigt eine schematische Darstellung eines Plasmaspritzgeräts, Bestandteil eines thermischen Spritzsystems entsprechend der Figur 6.

### Erfindungsgemässe Spritzpulver

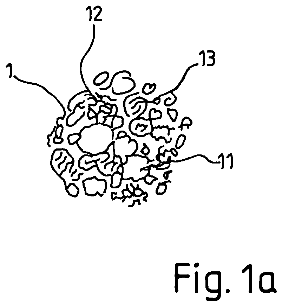

Für die Herstellung einer ersten Spritzpulvervariante zur Erzeugung der thermisch gespritzten Schicht werden erfindungsgemäss zunächst etwa 0.1 bis max. 5 µm grosse, in einem Fällungsverfahren hergestellte Kalziumphosphatpartikel, mit oder ohne antibakterielle Metall-Ionen (z.B. Ag) im Molekül oder auf Zwischengitterplätzen des Kristallgitters, mit antibakteriellen Metall-Partikeln (beispielsweise Ag) der gleichen Grössenordnung im benötigten Verhältnis gemäss Stand der Technik homogen gemischt, sprühgetrocknet, gegebenenfalls gesintert und in der gewünschten Kornverteilung fraktioniert. Es entstehen Metall/Kalziumphosphat-Spritzpulverkörner entsprechend Fig. 1a. Jedes Pulverkorn 1 enthält eine durch das Mischungsverhältnis festgelegte Anzahl von Metall-Partikeln (z.B. Ag) 12, statistisch und entsprechend ihrer Grössenverteilung im Verbund der verbackenen Kalziumphosphatpartikel 11 eingelagert. Die Ag-Partikelfraktion ist bevorzugt nach unten begrenzt, um sie so klar gegenüber atomar (als Ion) eingelagertem Ag abzugrenzen. Insbesondere werden Ag-Partikel 12 mit einem Durchmesser unter 0,1 µm ausgeschlossen. Bevorzugte Verwendung ist die Fraktion 0,5 bis 5 µm, für Sonderzwecke aber auch 1 bis 10 µm. Das bedeutet, dass sich auch Ag-Partikel 12 in den Spritzpulverkörner befinden, welche grösser sein können als die Kalziumphosphat-Partikel 11. Ein wichtiges Merkmal des erfindungsgemäss benötigten Metall-Kalziumphosphat-Spritzpulver (mit beispielsweise Ag) ist nun, dass für seine Herstellung zwei verschiedene Kalziumphosphat-Partikel benützt werden können. Einmal welche, die im vorherigen Fällungsverfahren in definierter Anzahl mit Metall-Ionen im Austausch gegen Kalzium-Ionen dotiert wurden 13, zum anderen auch mit Kalziumphosphat-Partikeln ohne diese Metall-Ionendotierung 11. Damit besteht die Möglichkeit, den Metall-Gehalt des Spritzpulvers in zwei Grössenebenen definiert festzulegen:
a) In der atomaren Grösse, als Metall (Ag)-Ionen im Kalziumphosphat-Gitter und/oder in den Zwischengitterplätzen des Kristallgitters eingebaut 13. Dabei wird z.B. der Ag-Gehalt in weiten Grenzen durch das Konzentrationsverhältnis des Ag-Salzes (beispielsweise Ag-Nitrat) in der wässrigen Lösung von z.B. Na-Phosphat und Ca-Clorid im jeweiligen Mischungsverhältnis zur Ausfällung der gewünschten Kalziumphosphatvariante festgelegt, unter Beachtung vorgeschriebener Reaktionszeiten und -temperaturen. Bevorzugt wird der Ag-Gehalt zwischen 0,05 und 2 % im Verhältnis zum Kalziumphosphat eingestellt. Als besonders wirksamer Bereich hat sich 800 ppm bis 2000 ppm erwiesen.
b) In der Grösse von konkreten Materialportionen als Metall (Ag)-Partikel 12, im Verbund mit den Kalziumphosphat-Partikeln 11 zum Spritzpulverkorn vereint. Der Metall-Gehalt in dieser Ebene kann z.B. mittels Grösse und Anzahl der im Mischprozess eingelagerten Ag-Partikel (12) in weiten Grenzen variiert werden. Als Bandbreite werden 0,1 bis 10 % empfohlen, bevorzugt auf 0.5 bis 2 % eingestellt.

Der Gesamt-Silbergehalt des Spritzpulvers darf einerseits nicht unterhalb der Konzentration liegen, ab welcher der Ag-Gehalt in der daraus entstandenen Spritzschicht nicht mehr antibakteriell wirkt. Gemäss Literaturangaben liegt diese Minimalkonzentration für jeden Patienten bei etwa 30 mg/kg Körpergewicht. Aufgabe der Erfindung ist ja, die Ag-Konzentration in der Wirkungsebene Schichtoberfläche/Knochenbett nach der zementlosen Implantation im benötigten Zeitraum permanent über einer wirksamen Minimalkonzentration zu halten. Auf der anderen Seite ist unbedingt sicherzustellen, dass der Ag-Gehalt in der Spritzschicht sicher genügend weit unterhalb der Toxizitätsgrenze für menschliche Gewebe zu liegen kommt. Auch hierzu finden sich einschlägige Literaturhinweise, welche je nach Testmethode und Konstitution des Patienten Werte zwischen 0,5 g bis 1,2 g/kg Körpergewicht empfehlen.

Erfindungsgemäss kann der Ag-Gesamtgehalt auch noch zusätzlich dadurch fein eingestellt werden, dass dem Ag-enthaltenden Kalziumphosphat-Spritzpulver noch in einem festgelegten Verhältnis Kalziumphosphat-Körner ohne Ag-Gehalt zugemischt werden. Ihre Grösse liegt innerhalb der Grenzen der Kornverteilung des Ag-enthaltenden Spritzpulvers. Als Mischungsverhältnis der Spritzpulveranteile mit und ohne Ag haben sich 90 bis 10 % bewährt, vorzugsweise 70 bis 30 %. Für Langzeitimplantate besser 40 bis 90 % und bei Spritzpulver für die Metall-enthaltenden Spritzschichten der Traumatologie besser 10 bis 40 %, wobei die prozentualen Angaben sich auf die Zumischung von Metall-freiem Kalziumphosphat beziehen. Diese Zumischung kann entweder vor dem Spritzen mit festgelegtem Mischungsverhältnis im Spritzpulver selbst oder später während der Schichtbildung über eine 2. Pulverförderlinie in den thermischen Energiefreistrahl erfolgen. Dann besteht sogar die zusätzliche Möglichkeit, dieses Mischungsverhältnis während der Schichtbildung zu verändern, um z.B. eine Schicht mit gradiertem Metall-Gehalt in Abhängigkeit der Schichtdicke zu erzeugen. Auch Sandwich-Strukturen lassen sich so spritzen, bei welchen jede Schichtlage einen definiert eingestellten Metall-Gehalt besitzt. Diese Varianten werden später noch genauer beschrieben, unter Bezug zur Anwendung der Metall-enthaltenden Kalziumphosphat-Spritzschichten in der Traumatologie und in der Orthopädie.

Um die erfindungsgemässe, antibakterielle Kalziumphosphatschicht durch thermisches Spritzen zu erzeugen, hat sich auch eine weitere Pulvervariante bewährt. Der Metall-Gehalt in atomarer Ebene durch den Einbau von Metall-Ionen im Kalziumphosphat-Kristallgitter und auf Zwischengitterplätzen erfolgt erst im fertigen Kalziumphosphat-Spritzpulver mit der für den thermischen Spritzprozess benötigten Kornverteilung und zwar mit Hilfe eines Absorptions- und Immobilisierungsprozesses in einer chemischen Lösung. So können z.B. 100 g fertiges Kalziumphosphat-Spritzpulver (mit oder ohne eingelagerte Ag-Partikel (12)) in wässriger Lösung von 10 g Ag-Nitrat in 1000 g destilliertem H₂O bei festgelegter Einwirkzeit und Badtemperatur mit der benötigten Anzahl von Ag-Ionen ergänzt werden. Als Kontrolle für die Ag-Konzentration im Pulver hat sich die Bestimmung des Gehaltes an Ca-Ionen in der Lösung bewährt, da diese ja durch die Ag-Ionen ersetzt und folglich freigesetzt wurden. Als besonders wirksame Metall-Konzentration bei dieser Pulvervariante haben sich 500 bis 2000 ppm Ag erwiesen, bevorzugt 1000 ppm.

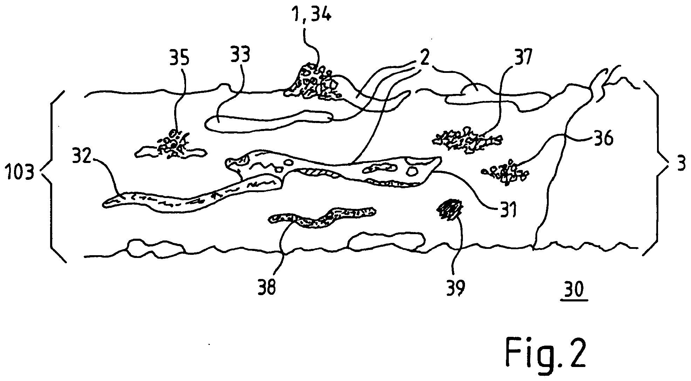

Es ist auch denkbar, Metall-Partikel ohne Beschränkung der Erfindung in der Grösse der Spritzpulverkörner in das Kalziumphosphat-Spritzpulver (Fig. 2) einzumischen, z.B. um es als reine Ag-Lamelle 38 in die Spritzschicht einzubauen. Die Grösse der Ag-Partikel ist aber vorzugsweise mit 50 µm nach oben begrenzt.

### Erfindungsgemässe Spritzschichten

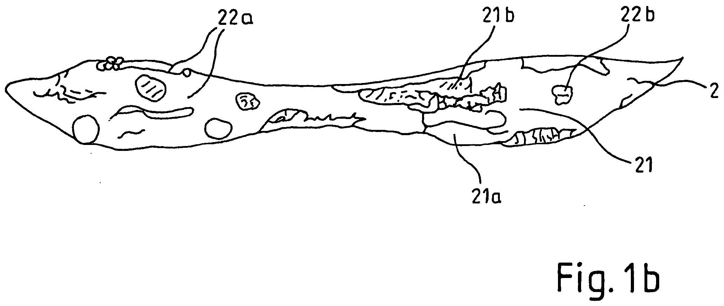

Werden nun die gemäss Beschreibung hergestellten Kalziumphosphat-Pulver durch thermisches Spritzen in Spritzschichten überführt, wird bei entsprechender Wahl der thermischen Energie jedes Spritzpulverkorn entsprechend Fig. 1a in eine Spritzlamelle (Fig. 1b) 2 umgewandelt, durch die mechanische Energie beim Aufprall auf der Substratoberfläche flach verformt und verbreitert. Die einzelnen Kalziumphosphat-Partikel 11 bilden einen homogenen Kern 21, in welchem die Metall-Partikel 12 je nach Grösse entweder als geschmolzene Feinlamellen 22a oder unverändert in der ursprünglichen Form 22b eingelagert sind, sich aber auch an ihrer freien Oberfläche befinden. Der homogene Kern 21 selbst setzt sich aus Metall-reichen 21b und Metall-freien 21a Zonen zusammen, je nachdem ob an der entsprechenden Stelle ein geschmolzenes Kalziumphosphat-Partikel mit atomarem Metall-Gehalt 13 oder ohne atomarem Metall-Gehalt 11 sich befindet.

Eine in Fig. 2 gezeigte Spritzschicht 3 (bzw. Deckschicht 103), welche auf einem Implantat 30 liegt, setzt sich aus einer Vielzahl einzelner Spritzlamellen 2 zusammen, wobei die Energie des Spritzprozesses wahlweise auch so eingestellt werden kann, dass ein festgelegter Anteil des Metall-Kalziumphosphat-Pulvers ohne Umwandlung in eine Spritzlamelle, also in seiner Ausgangsform als Pulverkorn 1 in die Spritzschicht 3 eingelagert wird. Bevorzugt betrifft das die grösseren Spritzpulverpartikel der gewählten Pulverfraktion. Insgesamt können die erfindungsgemässen Spritzschichten je nach Wahl der thermischen Spritzenergie und Form des Ausgangspulvers folgende Komponenten in unterschiedlicher Konzentration und in verschiedenen Ebenen angeordnet enthalten:
a) Geschmolzene Spritzlamellen 31 (Wirklamelle) mit eingeschlossenen, geschmolzenen Metall-Feinlamellen 22a und ungeschmolzenen Metall-Partikeln 22b, wobei der Kalziumphosphatanteil 21 einerseits Metall-Ionen enthält 21b, andererseits Metall-frei ist 21a.
b) Geschmolzene Spritzlamellen 32 (Wirklamelle) entsprechend a)mit Metall-Ionen im Kalziumphosphatanteil 21b.
c) Geschmolzene Spritzlamellen 33, welche weder Metall-Ionen noch konkrete Metall-Materialanteile in der Spritzlamelle enthalten. Sie werden als Fülllamellen bezeichnet, aus Füllkörnern entstanden.
d) Ungeschmolzene Wirkkörner 34, welche in ihrer Struktur und Zusammensetzung dem Ausgangspulver 1 entsprechen, also Metall-Partikel 12, Metall-Ionen im Kalziumphosphatanteil 13 und Metall-freies Kalziumphosphat 11 enthalten.
e) Ungeschmolzene Wirkkörner 35 gemäss d) ohne Metall-Partikel 12 dafür aber mit Metall-Ionen im Kalziumphosphatanteil 21.
f) Ungeschmolzene Wirkkörner 36 gemäss d) mit Metall-Partikel 12 dafür ohne Metall-Ionen im Kalziumphosphatanteil 21.
g) Ungeschmolzene Füllpartikel 37 ohne Ag, weder atomar noch als konkrete Materialportion.

Für bestimmte Anwendungen der Metall-enthaltenden, thermisch gespritzten Kalziumphosphat-Schicht kann es vorteilhaft sein, nur die atomare Form der Metall-Einlagerung in jedes einzelne Korn des Spritzpulvers durch Ionen-Austausch einzubringen. Die Spritzlamellen entsprechen dann denen der reinen Kalziumphosphat-Schicht, mit dem einzigen Unterschied, dass wie im Spritzpulver in jeder Spritzlamelle einzelne Ca-Ionen durch Metall-Ionen ersetzt und oder diese auf Zwischengitterplätzen im Kalziumphosphatgitter eingebaut sind.

Die Metall-Zugabe in Form konkreter Materialportionen erfolgt durch Metall-Spritzpulverkörner, in der Grösse dem unteren Bereich der Kalziumphosphat-Spritzpulverkornverteilung entsprechend. Beträgt diese z.B. 20 bis 50 µm, so wird die Metall-Kornfraktion bevorzugt 5 bis 25 µm gewählt. Dann entsteht eine Metall-Kalziumphosphat-Schicht 3 mit grossflächigen Metall-Spritzlamellen (38), z.B. Ag-Lamellen.
Dabei ist allerdings zu beachten, dass in der Spritzschicht grossflächige, reine Metall-Bereiche präsent sind, je nach Korngrösse im Durchmesser bis zu 100 µm gross und etwa 0,2 bis 5 µm dick, bevorzugt 20 µm gross und 1 µm dick. Ein Teil der Metallkörner kann auch als ungeschmolzene Metallkörner (39) in die Kalziumphosphatschicht 3 eingelagert sein.

Bei der Metall-Zugabe während der Spritzpulverherstellung ist zu beachten, dass der thermische Spritzprozess zu Veränderungen der Verteilungsverhältnisse zwischen Spritzpulver und Spritzschicht führen kann, vor allem dann, wenn das Spritzpulver zur Erzeugung einer dichten Schichtstruktur gut aufgeschmolzen wird. Diesen Effekt kann man zum Teil dadurch kompensieren, dass die im Schmelzzustand einen höheren Dampfdruck entwickelnde Komponente beim Mischen in einer Kornverteilung eingebracht wird, deren mittleren Partikelgrösse zu grösseren Partikeln verschoben ist. Vergleicht man hierzu Kalziumphosphat (keramischer Werkstoff) z.B. mit Silber (Metall) so ist aufgrund der physikalischen Kenndaten (Schmelz- und Verdampfungstemperatur, Wärmeleitfähigkeit und Wärmekapazität) eher mit einer Erniedrigung des atomaren Ag-Gehaltes und mit einer Erhöhung des Ag-Gehaltes in Form der konkreten Materialportionen in der Spritzschicht im Vergleich zum Mischungsverhältnis im Spritzpulver zu rechnen. Das prozentuale Konzentrationsverhältnis Ag/Kalziumphosphat im Spritzpulver wird sich folglich in der Spritzschicht entweder zugunsten von Ag erhöhen oder erniedrigen, je nachdem ob der Ag-Anteil in konkreten Materialportionen im Spritzpulver vorliegt, oder als atomarer (ionischer) Ag-Anteil.

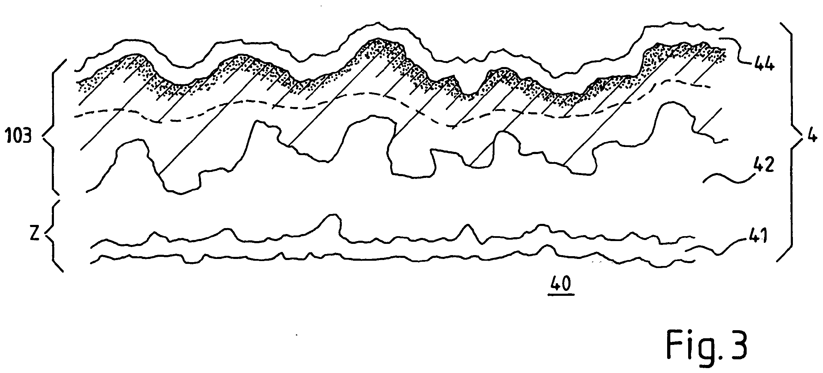

Weitere Varianten zur erfindungsgemässen Herstellung der entzündungshemmenden, osseoinduktiven und antibakterielles Metall in definierter Form und Konzentration enthaltenden Kalziumphosphatschicht berücksichtigen wichtige Erkenntnisse aus der medizintechnischen Anwendung der thermischen Spritztechnik und werden mit Hilfe der Fig. 3 näher erläutert, welche eine Schichtfolge 4 auf einem für die Orthopädie vorgesehenen Implantat 40 zeigt.

Auf diesem Implantat 40 wird in vorteilhafter Weise zunächst die Haftkraft der erfindungsgemässen z.B. im VPS-Verfahren als Deckschicht aufgespritzten Metall/Kalziumphosphat-Schicht 103 durch eine erste Spritzlage aus Titan 41, etwa 20 bis maximal 50 µm dick, als Haftschicht wirkend, gesichert. Titan ist ein bekannter Haftvermittler und verankert sich sehr gut mit allen Oberflächen der für die Herstellung von Endoprothesen verwendeten Materialien: Metalle (z.B. Ti), Metalllegierungen z.B. CoCr, Kunststoffe z.B. PEEK ohne oder mit Kohlefaserverstärkung und Keramiken z.B. Al₂O₃, ZrO₂ und Mischkeramiken. Dabei werden alle Implantatwerkstoffe vor der Beschichtung bevorzugt im Sandstrahlverfahren aufgerauht, um den Verankerungseffekt zu verstärken. Diese Ti-Haftschicht 41 ist für Keramikprothesen zwingend nötig, nur eine Aufrauhung der Oberfläche im Sandstrahlverfahren erzeugt nicht die benötigte Haftkraft. Für Implantate aus Edelstahl darf keine Ti-Haftschicht verwendet werden. Um die erfindungsgemässen Metall/KalziumphosphatSchichten 103 auch auf weniger biokompatible Werkstoffe aufbringen zu können, z.B. auf kohlefaserverstärktes PEEK, wirkt die zunächst nur im Sinne der Haftvermittlung aufgespritzte Ti-Schicht 41 gleichzeitig als Versiegelung der Substratoberfläche und erzwingt damit ihre Umwandlung in ein biokompatibles Implantat.

Ohne Unterbrechung des Beschichtungsvorganges wird die gesamte Schichtfolge der Metall/HA-Schicht 103 direkt auf die frisch gespritzte Ti-Unterlage (41) aufgebracht. Dabei wird die Energie des Plasmafreistrahles, in den das erfindungsgemäss hergestellte Metall/HA-Wirkpulver injektiert wird, wahlweise so eingestellt, dass entweder die Pulverpartikel vollständig und/oder nur teilweise an- oder aufgeschmolzen werden. Dieser so gezielt gesteuerte Spritzprozess ermöglicht folglich, die Metall/HA-Schicht wahlweise mit hoher Kristallinität oder mit hohem amorphem Strukturanteil herzustellen. Für bestimmte Anwendungen kann es auch vorteilhaft sein, diesen Übergang von kristalliner zur amorphen Schichtstruktur gradiert zu gestalten. Das bedeutet, dass während des Spritzprozesses bei kontinuierlicher Pulverinjektion laufend die Plasmafreistrahlenergie von zum Beispiel niedrigeren Werten zu höheren Werten verschoben wird, die Schicht folglich in Richtung Schichtoberfläche in ihrer Struktur immer dichter, gleichzeitig aber auch mehr amorph wird. Aus der Literatur ist nämlich bekannt, dass die Löslichkeit von HA, umgeben von menschlichem Körpergewebe, mit zunehmendem amorphem Schichtanteil steigt. Damit erhält die erfindungsgemäss so hergestellte Metall/HA-Schicht 103 eine gradierte Löslichkeit, welche in der Anfangsphase unmittelbar nach der Implantation höher ist und mit zunehmender Verweilzeit im Körper sich kontinuierlich reduziert. So wird einerseits das Verwachsen der Prothesenoberfläche durch die gelösten Kalzium- und PhosphatIonen unterstützt und beschleunigt. Auf der anderen Seite ist auch die Metall-Ionenabgabe anfangs erhöht und folglich die antibakterielle Wirkung in der kritischen Phase unmittelbar nach der Implantation verstärkt. Mit zunehmender Abdeckung der Implantatoberfläche durch neu gebildetes Knochengewebe sinkt der Bedarf an antibakteriellem Metall zur sicheren Vermeidung einer Infektion. Dieser Effekt variabler (zu- oder abnehmender) Metall-Konzentration im Knochen/Prothesenverbund in Abhängigkeit von der Verweilzeit kann zusätzlich auch dadurch gesichert werden, dass gleichzeitig mit der Erhöhung der thermischen Energie im Plasmafreistrahl die Zumischung von Metall-freiem Kalziumphosphat-Spritzpulver mit Hilfe einer zusätzlichen Pulverinjektion entweder in Stufen (Schicht 103 als Sandwich-Struktur wie in Fig. 3 gezeigt) oder kontinuierlich (Schicht 103 mit gradierte Metall-Konzentration nicht schematisch dargestellt) geändert wird. Ausdrücklich darauf hingewiesen wird an dieser Stelle, dass die Ausbildung der geschilderten Schichtvarianten nicht auf das Plasmaspritzen beschränkt ist. Auch mit den übrigen Verfahren der thermischen Spritztechnik können Freistrahlenergie und zusätzliche Pulverinjektionen entsprechend variiert und zugeschaltet werden.

Zur generellen Unterstützung der osseokonduktiven und osseoinduktiven Wirkung der Metall/Kalziumphosphat-Spritzschicht kann zwischen die Titanhaftschicht 41 und die z.B. Ag/HA-Schicht 103 auch noch eine rauhe Ti-Schichtstruktur 42 aufgespritzt werden. Somit liegt im Ausführungsbeispiel gemäß der Figur 3 eine aus 2 Lagen bestehende Zwischenschicht Z vor, die aus der Titanhaftschicht 41 und der rauen Ti-Schichtstruktur 42 besteht. Dabei wird die Kornfraktion des benötigten Ti-Spritzpulvers so gewählt, dass die Schichtoberfläche eine rauhe, offenporige Konfiguration erhält, welche besonders günstig ist für das Einwachsen der Knochenzellen. Diese bevorzugen eine offene Oberflächenporosität mit Poren im Bereich zwischen 50 und 400 µm. Der Trick dabei ist, dass sich diese Oberflächenstruktur der Ti-Zwischenlage 42 in die darauf abgeschiedene Metall/Kalziumphosphat-Schicht 103 überträgt, also nur unwesentlich, allerdings mit zunehmender Dicke der Metall/Kalziumphosphat-Schicht 103 stärker eingeebnet wird. Insbesondere in dieser Schichtvariante wird deshalb die Schichtdicke der Metall/Kalziumphosphat-Schicht 103 auf maximal 150 µm beschränkt und bevorzugt auf 30 bis 100 µm eingestellt. Im Fall der gradierten Kristallinität sollten beispielsweise die ersten 20 bis 60 µm der antibakteriellen Kalziumphosphatschicht hochkristallin sein. Im mittleren Schichtbereich von 50 bis 120 µm sollte dann der amorphe Anteil stetig zunehmen und in der letzten, oberflächlichen und etwa 20 bis maximal 50 µm dicken Lage dann am höchsten sein, z.B. mindestens 40 bis 80 % betragen, bevorzugt 55 bis 70 %.

Ohne Einschränkung der Erfindung ist es auch möglich, auf die erfindungsgemäss hergestellte Metall/Kalziumphosphat-Schicht 103 noch eine zusätzliche Metall-freie Kalziumphosphatschicht 44 hoher Löslichkeit aufzuspritzen, z.B. eine TCP-Schicht, eine hochamorphe HA-Schicht oder eine HA/TCP-Mischschicht, in der Dicke auf 10 bis 60 µm beschränkt, bevorzugt etwa 20 bis 40 µm dick, da bei diesem Wert auch bei thermisch gespritzten Schichten schon eine vollständig geschlossene Abdeckung entsteht. Für das HA/TCP Mischungsverhältnis hat sich der Bereich 10/90 bis 40/60 % bewährt, wobei der TCP-Anteil bevorzugt höher bei 60 bis 80 % liegt, wenn eine dünne, nur etwa 10 bis 20 µm starke Abdeckschicht (44) verwendet wird. Somit liegt im Ausführungsbeispiel gemäß der Figur 3 eine mehrschichtige Spritzschicht 4 vor, die aus der mehrlagigen Metall/Kalziumphosphat-Deckschicht 103 wahlweise mit gradierter oder Sandwich-Struktur, der Abdeckschicht 44 und der ein- oder zwei-lagigen Zwischenschicht Z (Haftschicht 41 und Ti-Struktur 42) besteht.

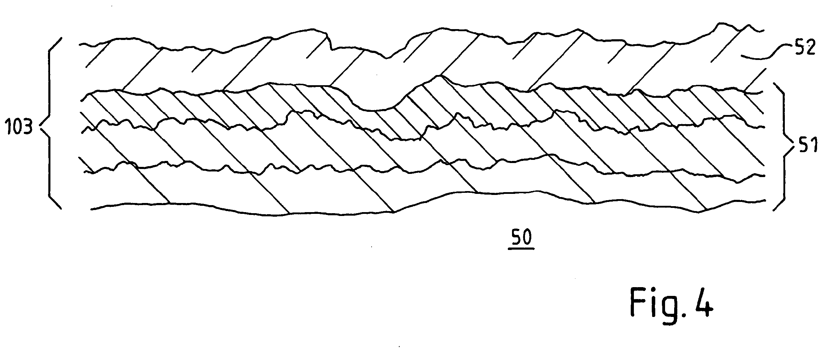

Fig. 4 zeigt ein weiteres Beispiel einer erfindungsgemässen Deckschicht (103), welche im mehrlagigem Aufbau für den Anwendungsfall Traumatologie optimiert ist. Sie besteht aus einer 1. Lage Kalziumphosphat 51 mit variablem amorphen Strukturanteil und TCP-Gehalt direkt auf der Oberfläche eines Implantates 50 aufgespritzt, entweder als Sandwich-Struktur oder als gradierte Schichtfolge aufgebaut, mit mindestens 10 bis 40 % TCP und 40 bis 90 % HA mit einem amorphen Schichtanteil von wenigstens 20 bis 80 %, bevorzugt 50 bis 60 %. Die Dicke dieser 1. Lage 51 kann wahlweise 20 bis 100 µm betragen, bevorzugt 40 bis 60 µm. Beim Spritzen dieser 1. Lage 51 wurde die thermische Energie so gewählt, dass etwa 70 bis 100 % der Spritzpulverkörner in geschmolzene Spritzlamellen umgewandelt wurden, bevorzugt etwa 80 bis 90 %. Dabei wird wieder die Energie des Plasmafreistrahles variiert, im Gegensatz zur Schichtfolge 4 für die Orthopädie aber im Anwendungsfall Traumatologie so, dass der kristalline Schichtanteil in Richtung Substratoberfläche abnimmt. Für diese 1. Lage 51 wurde ein Metall-Kalziumphosphat-Pulver mit einem Metall-Gehalt gewählt, welcher nur leicht über der antibakteriellen Wirkungsgrenze von 300 ppm liegt, bevorzugt 500 bis 1150 ppm. Über dieser 1. Lage 51 befindet sich eine 2. Lage 52, nur etwa 10 bis 50 µm, bevorzugt 20 bis 30 µm dick. Sie zeichnet sich gegenüber der 1. Lage durch einen höheren Metall-Gehalt bei gleichzeitig geringerer Löslichkeit aus. Idealerweise benötigt das aufgrund der osseokonduktiven/osseoinduktiven Wirkung sehr rasch auf der Beschichtung ausgebildete neue Knochengewebe etwa 3/4 der vorgesehenen Implantationszeit, um diese 2. Lage 52 der Gesamtbeschichtung aufzulösen und/oder in weiteres Knochengewebe umzuwandeln. Danach steht das neue Knochengewebe im direkten Kontakt zu der noch vorhandenen 1. Lage 51 der Metall/Kalziumphosphat-Spritzschichtfolge. Diese wird nun innerhalb des restlichen 1/4 der Implantationszeit sehr rasch ebenfalls aufgelöst und/oder in Knochengewebe umgewandelt. Verantwortlich hierfür ist der in Richtung Prothesenoberfläche zunehmende Gehalt an TCP und/oder amorphem HA der 1. Lage 51, welcher eine ansteigende Löslichkeit bewirkt. Als Folge davon kommt das neu gebildete Knochengewebe in den direkten Kontakt zur Prothesenoberfläche, welche glatt ist und eine mittlere Rauhigkeit von deutlich unter 2 µm, bevorzugt 0,1 bis 1 µm aufweist. Bei dieser Oberflächenkonfiguration bildet sich eine Bindegewebszone zwischen dem Knochengewebe und der Prothesenoberfläche, welche die vorgesehene Explantation dieser Traumaprothese nach der vorgesehenen Verweilzeit und dem Abschluss der Ausheilung des Knochenbruches erleichtert. Gemäß dem in der Figur 4 gezeigten Ausführungsbeispiel ist somit eine Deckschicht 103 Zwischenschicht (Haftschicht und/oder Ti-Struktur) direkt auf dem Implantat 50 aufgespritzt.

### Ausführungsbeispiele der Erfindung

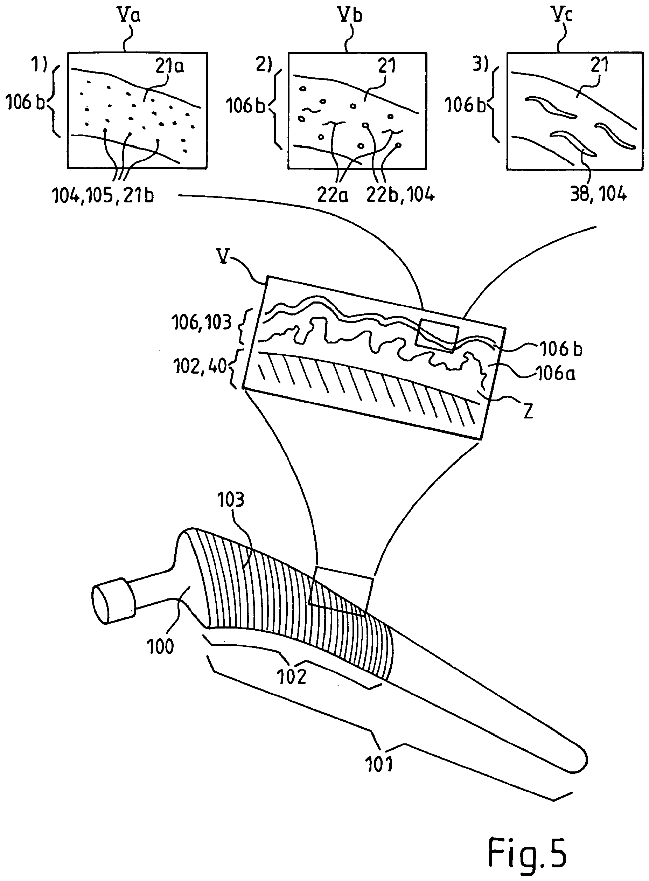

In Fig. 5 ist als ein praktisches Ausführungsbeispiel für ein beschichtetes Implantat der Anwendung Orthopädie ein Hüftschaft 100 gezeigt, üblicherweise aus einer Titanlegierung hergestellt. Teil 101 dient als Verankerungsbereich in einem nicht dargestellten Oberschenkelknochen. Dieser Verankerungsbereich des Hüftschaftes ist entweder komplett, oder wie in Fig. 5 gezeigt, in einem Teilbereich 102 mit der mittels thermischer Spritztechnik hergestellten Deckschicht 103 beschichtet. Im Teilbereich 102 bildet folglich der Hüftschaft 100 die Substratoberfläche 40 für die Deckschicht 103, im dargestellten Beispiel mit Zwischenschicht Z (Haftschicht 41 und zusätzlicher Ti-Schichtstruktur 42) aufgespritzt. Die Haftschicht 41 bildet gemeinsam mit der Ti-Schichtstruktur 42 eine zweilagige Zwischenschicht Z. Der Eindeutigkeit wegen wird erwähnt, dass die Zwischenschicht Z unter der Deckschicht 103 und auf dem Substratmaterial 40 liegt. In der Figur 5 zeigt ein Fenster V einen prinzipiellen Aufbau der Deckschicht 103, welche auf der Zwischenschicht Z liegt, in vergrößerter Darstellung. Entsprechend der Darstellung in dem Fenster V folgt auf das Substratmaterial 40 die zweilagige Zwischenschicht Z aus Titan. Diese Zwischenschicht Z aus Titan ist, wie oben bereits erwähnt, nicht zwingend vorgesehen, findet aber immer dann Verwendung, wenn ein optimiertes Anhaften der nachfolgend aufgebrachten Kalziumphosphatschicht 103 gewünscht ist bzw. wenn eine erhöhte Rauhigkeit gegenüber der nachfolgend aufgebrachten Kalziumphosphatschicht 103 gewünscht ist. Die nachfolgend aufgebrachte Kalziumphosphatschicht 106 (entsprechend 103) ist gemäß einer ersten Ausführungsvariante durch eine Kalziumphosphatschicht 106a ohne antibakterielle Wirkung und eine auf diese folgende Kalziumphosphatschicht 106b mit Wirkbestandteilen 104 gegen Bakterien ausgebildet. Gemäß einer zweiten, nicht dargestellten Ausführungsvariante besteht die Kalziumphosphatschicht 103 ausschließlich aus einer Kalziumphosphatschicht 106b mit Wirkbestandteilen 104 gegen Bakterien, im inneren Aufbau folglich der Fig. 3 entsprechend. Generell sind für den Aufbau der Deckschicht 103 z.B. folgende Varianten vorgesehen:
Variante 1: Die Deckschicht 103 besteht aus einer Kalziumphosphatschicht 106a ohne antibakterielle Wirkung und einer Kalziumphosphatschicht 106b mit Wirkbestandteilen 104 gegen Bakterien, wobei die Kalziumphosphatschicht 106b die äußerste, mit dem Knochen bzw. dem Gewebe in Kontakt kommende Schicht darstellt.
Variante 2: Die Deckschicht 103 besteht nur aus einer Kalziumphosphatschicht 106b mit Wirkbestandteilen 104 gegen Bakterien, wobei die Kalziumphosphatschicht 106b ebenfalls die äußerstes mit dem Knochen bzw. dem Gewebe in Kontakt kommende Schicht darstellt.
Variante 3: Die Deckschicht 103 besteht aus einer ersten Lage 106a ohne antibakterielle Wirkung, aus einer 2. Lage 106b mit antibakteriellen Wirkung, gefolgt von einer weiteren Kalziumphosphatschicht 44 ohne antibakteriellen Wirkung, dafür mit hoher Löslichkeit ausgebildet.

In den Fenstern Va bis Vc sind nun drei Beispiele für den Aufbau der Kalziumphosphatschicht 106b mit Wirkbestandteilen 104 gegen Bakterien schematisch dargestellt.

Gemäß einer ersten Ausführungsvariante Va ist die Kalziumphosphatschicht 106b so aufgebaut, dass die Wirkbestandteile 104 als Metall-Ionen 105 (vergrössert dargestellt) in Kalziumphosphat 21b eingelagert sind, welches gemeinsam mit dem metallfreiem Kalziumphosphat 21a die Schicht bilden. Hierbei liegen die Metall-Ionen 105 zum Beispiel als Ag⁺-Ionen und/oder als Cu²⁺-Ionen vor, oder als Mischungen beider.

Gemäß einer zweiten Ausführungsvariante Vb ist die Kalziumphosphatschicht 106b so aufgebaut, dass Metallpartikel 22a und/oder 22b, welche die Wirkbestandteile 104 bilden, homogen verteilt in Kalziumphosphat 21 eingelagert sind. Hierbei liegen die geschmolzenen und/oder ungeschmolzenen Wirkbestandteile 104 zum Beispiel als Ag-Partikel und/oder als Cu-Partikel vor.

Gemäß einer dritten Ausführungsvariante Vc ist die Kalziumphosphatschicht 106b so aufgebaut, dass Lamellen 38 aus Metall die Wirkbestandteile 104 bilden, welche in Kalziumphosphat 21 eingelagert sind. Hierbei bestehen diese antibakteriellen Metall-Lamellen 38 zum Beispiel aus Ag und/oder Cu oder aus Zn oder aus Mischungen dieser antibakteriellen Metalle.

Gemäß den oben stehenden Ausführungen und den detaillierten Angaben zur erfindungsgemässen Herstellung der Spritzpulver und Spritzschichten sind als weitere Ausführungsvarianten auch Mischformen der Ausführungsvarianten Va bis Vc vorgesehen, z.B. welche bei denen sich die Struktur der Kalziumphosphatschicht 106b in Richtung Substratmaterial 40 hin verändert, indem sich beispielsweise die Form, in welcher die Wirkbestandteile 104 vorliegen, verändert und/oder wobei die Konzentration der Wirkbestandteile 104 zunimmt oder abnimmt.

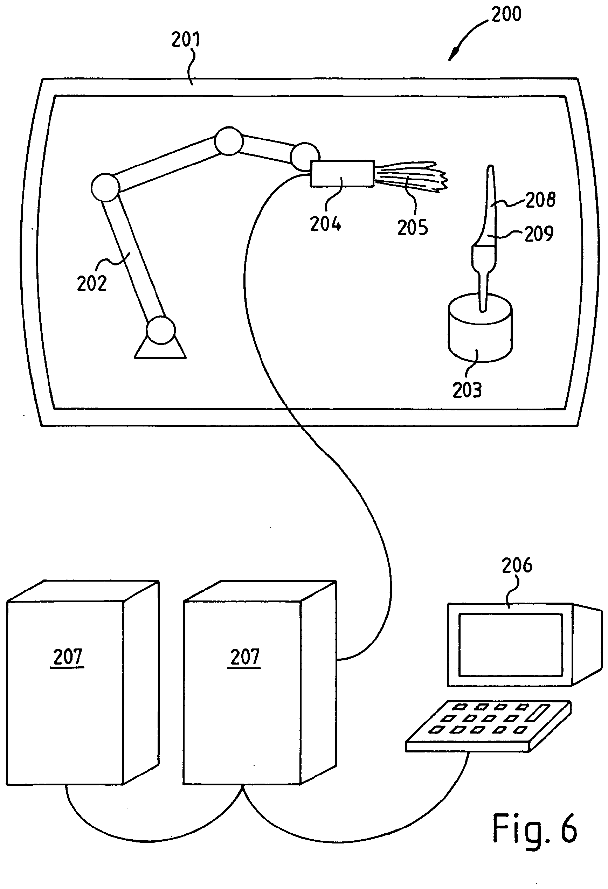

In der Figur 6 ist in schematischer Darstellung ein thermisches Spritzsystem 200 gezeigt, welches zur Herstellung eines erfindungsgemäßen Implantats Verwendung findet bzw. mit welchem das erfindungsgemäße Verfahren durchführbar ist. Das Spritzsystem 200 umfasst eine Vakuumkammer oder eine Schallschutzkabine 201, in welcher ein Roboter 202 und eine Haltevorrichtung 203 angeordnet sind. Der Roboter 202 führt einen Brenner 204, in welchem ein Plasmastrahl 205 erzeugbar ist. Das Spritzsystem 200 umfasst weiterhin eine Bedieneinheit 206, mit welcher insbesondere der Roboter 202 und der Brenner 204 steuer- und regelbar sind. Weiterhin umfasst das Spritzsystem 200 noch die üblichen Peripheriegeräte 207 wie zum Beispiel Kühlsystem, Schaltschrank, Behälter für Spritzmaterial und ähnliches. In der Haltevorrichtung 203 ist ein unbeschichtetes Implantat 208 gehalten, welches nach dem Aufspritzen einer speziellen Deckschicht als erfindungsgemäß beschichtetes Implantat 209 dem Spritzsystem 200 entnommen werden kann.

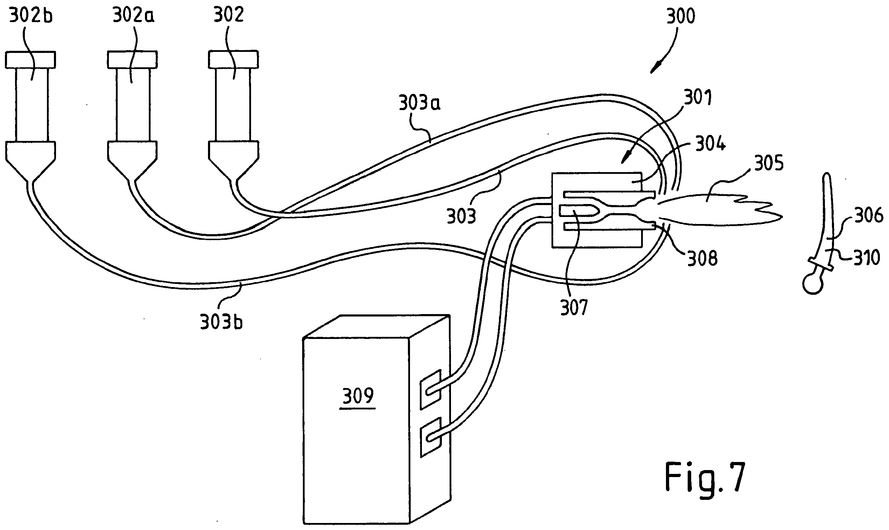

In der Figur 7 ist ein Plasmaspritzgerät 300 schematisch dargestellt, welches zum Beispiel in dem in der Figur 6 dargestellten thermischen Spritzsystem verwendbar ist. Das Plasmaspritzgerät 300 umfasst einen Brenner 301, welcher auch als Plasmatron bezeichnet wird. Diesem wird über einen oder mehreren Pulverförderer 302, 302a und 302b erfindungsgemäß zusammengesetzte Pulver 303, 303a und 303b zugeführt, und mit dem aus einer Düse 304 austretenden Plasmastrahl 305 auf dem zu beschichtenden Implantat 306 abgeschieden. Die Düse 304 umfasst im Wesentlichen eine Kathode 307 und eine Anode 308. Der Brenner 301 ist zur Stromversorgung und Kühlung an ein Versorgungsmodul 309 angeschlossen. Nach einer Plasmabeschichtung mit den entsprechenden Pulvern 303, 303a und 303b liegen dann die erfindungsgemäß hergestellten Implantate 310 mit antibakteriellen Eigenschaften vor, wobei die Spritzpulver einzeln nacheinander oder gleichzeitig mit variablem Anteil in den Plasmafreistrahl injektiert werden.

### Bezugszeichenliste:

- 1: Pulverkorn
- 2: Spritzlamelle
- 3: Spritzschicht
- 4: Schichtfolge

- 11: Kalziumphosphatpartikel
- 12: Ag-Partikel
- 13: Kalziumphosphatpartikel mit Ag-Ionen

- 21: homogener Kern, Kalziumphosphatanteil
- 21a: Ag-freie Kalziumphosphatzone
- 21b: Ag-reiche Kalziumphosphatzone
- 22a: geschmolzene Feinlamelle aus Ag
- 22b: ungeschmolzenes Ag-Partikel in ursprünglicher Form

- 30: Implantat
- 31: geschmolzene Spritzlamelle (1.Variante)
- 32: geschmolzene Spritzlamelle (2.Variante)
- 33: geschmolzene Spritzlamelle (3.Variante)
- 34: ungeschmolzenes Spritzpulverkorn (Wirkkorn) (1.Variante)
- 35: ungeschmolzenes Spritzpulverkorn (Wirkkorn) (2.Variante)
- 36: ungeschmolzenes Spritzpulverkorn (Wirkkorn) (3.Variante)
- 37: ungeschmolzenes Spritzpulverkorn (Füllkorn)
- 38: geschmolzene Ag-Spritzlamelle
- 39: ungeschmolzenes Ag-Spritzpulverkorn

- 40: Implantat
- 41: Ti-Haftschicht
- 42: rauhe Ti-Schichtstruktur
- 44: Ag-freie Kalziumphosphatschicht
- Z: Zwischenschicht, bestehend nur aus 41, oder aus 41 und 42

- 50: Implantat
- 51: 1. Lage
- 52: 2. Lage

- 100: Implantat
- 101: Verankerungsbereich
- 102: Beschichteter Verankerungsbereich
- 103: Deckschicht
- 104: Wirkbestandteil
- 105: Metallionen, z.B. Ag⁺,Cu²⁺oder Zn²⁺
- 106: Kalziumphosphatschicht
- 106a: Kalziumphosphatschicht ohne antibakterielle Wirkung
- 106b: Kalziumphosphatschicht mit Wirkbestandteilen

- 200: thermisches Spritzsystem
- 201: Vakuumkammer oder Lärmschutzkabine
- 202: Roboter
- 203: Haltevorrichtung
- 204: Brenner
- 205: Plasmastrahl
- 206: Bedieneinheit
- 207: Peripheriegeräte
- 208: unbeschichtetes Implantat
- 209: beschichtetes Implantat

- 300: Plasmaspritzgerät
- 301: Brenner, Plasmatron
- 302,a,b: Spritzpulver im Pulverförderer
- 303,a,b: Pulverleitungen
- 304: Düse
- 304: Plasmastrahl
- 306: noch zu beschichtendes Implantat
- 307: Kathode
- 308: Anode
- 309: Versorgungsmodul
- 310: Implantat mit antibakteriellen Eigenschaften

- V: Fenster
- Va-Vc: Fenster

## Patentansprüche

1. Zementlos implantierbare Prothese für die Traumatologie und/oder Orthopädie (40, 50, 100, 209, 310) aus biokompatiblen Materialien, welche einen Grundkörper (100) mit einem sich im Knochen oder Gewebe verankernden Verankerungsbereich (101) aufweist, wobei der Verankerungsbereich (101) zumindest bereichsweise mit einer Deckschicht (103) versehen ist, wobei die Deckschicht (103) aus einem Pulver (302) unter Anwendung eines thermischen Spritzverfahrens bevorzugt durch ein Plasmaspritzverfahrens gebildet ist, **dadurch gekennzeichnet, dass** das Pulver (302) im wesentlichen aus Kalziumphosphat besteht und antibakteriell wirksame Wirkbestandteile (104) umfasst und vorzugsweise die für einen Kontakt mit dem Knochen oder dem Gewebe vorgesehene Deckschicht (103) des Verankerungsbereichs (101) entweder direkt auf den Grundkörper (100) oder unter Einbau einer wenigstens einschichtigen Zwischenschicht (Z) auf den Grundkörper (100) insbesondere in einem Bereich (102) aufgebracht ist.

2. Implantat (40, 50, 100, 209, 310) nach vorhergehenden Anspruch, **dadurch gekennzeichnet, dass** die antibakteriell wirksamen Wirkbestandteile (104) der Deckschicht (103) aus Metall und/oder Edelmetall bestehen, bevorzugt aus Silber (12, 13) und/oder Kupfer und/oder Zink und Mischungen daraus umfassen, wobei die Wirkbestandteile (104) in atomarer und/oder ionischer und/oder kristallgitterintegrierter Form und/oder als konkrete Materialportionen vorliegen können und die Freisetzung der antibakteriell wirksamen Metallionen über die Löslichkeit des Kalziumphosphates beeinflussbar ist.

3. Implantat (40, 50, 100, 209, 310) nach einem der vorhergehenden Ansprüche, **dadurch gekennzeichnet, dass** die Wirkbestandteile (104) in konkreten Materialportionen vorzugsweise entweder ungeschmolzen als Partikel (12, 22b) bzw. als Spritzpulverkorn (34, 35, 36, 39) mit einem Durchmesser von 0,5 bis bevorzugt 25 Mikrometer und/oder als geschmolzene Spritzlamellen (22a, 31, 32, 38) in die Deckschicht (103) eingebaut sind.

4. Implantat (40, 50, 100, 209, 310) nach einem der vorangegangenen Ansprüche, **dadurch gekennzeichnet, dass** das Pulver (302) für die Herstellung der Deckschicht (103) im wesentlichen aus Kalziumphosphat, insbesondere aus Hydroxylapatit besteht und einen definierten Anteil Wirkbestandteil (104) aus Metall und/oder Edelmetall enthält, vorzugsweise Silber und/oder Kupfer und/oder Zink und/oder Mischungen daraus, wobei der Metallgehalt in atomarer und/oder ionischer Form vorliegt und/oder als konkrete Materialportion eingemischt wird.

5. Implantat (40, 50, 100, 209, 310) nach einem der vorangegangenen Ansprüche, **dadurch gekennzeichnet, dass** im Pulver (302) eine festgelegte Anzahl Ca-Ionen im Kristallgitter des Kalziumphosphates über einen Ionen-Austausch durch Metallionen, insbesondere Ag-Ionen ersetzt und/oder Metallatome auf Zwischengitterplätzen eingelagert sind.

6. Implantat (40, 50, 100, 209, 310) nach einem der vorangegangenen Ansprüche, **dadurch gekennzeichnet, dass** der Ionen-Austausch und/oder die Einlagerung der Metalle während der Ausfällung des gewünschten Kalziumphosphates aus wässriger Lösung durch Zugabe von Metallsalzen in einem festgelegten Verhältnis zu den für die Ausfällung des Kalziumphosphates benötigten Komponenten erfolgt, wobei die Anzahl der eingebauten Metall-Ionen und/oder eingelagerten Metallatome unter Beachtung vorgegebener Reaktionszeiten und -temperaturen bestimmt wird.

7. Implantat (40, 50, 100, 209, 310) nach einem der vorangegangenen Ansprüche, **dadurch gekennzeichnet, dass** der Ionen-Austausch und/oder die Einlagerung der Metallatome am fertigen Kalziumphosphatspritzpulverkörnern vorgenommen wird, welches in der optimierten Verteilung für das gewählte thermische Spritzverfahren zur Herstellung der Deckschicht (103) vorliegen.

8. Implantat (40, 50, 100, 209, 310) nach einem der vorangegangenen Ansprüche, **dadurch gekennzeichnet, dass** das Kalziumphosphat-Spritzpulver bestehend aus Füllkörnern (37) und/oder Wirkkörnern (34, 35, 36) mit Metallkörnern gemischt ist, welche der Kornverteilung des Kalziumphosphatpulvers angepasst sind und vorzugsweise nicht kleiner als 5 µm und nicht grösser als 25 µm ausgebildet sind.

9. Verfahren zur Beschichtung einer zementlos implantierbaren Prothese für die Traumatologie und/oder Orthopädie (40, 50, 100, 209, 310) oder eines Teiles davon, mit einer antibakteriell wirksamen Deckschicht (103), aus einem Pulver (302) unter Anwendung eines thermischen Spritzverfahrens, welches vorzugsweise als Plasmaspritzverfahren ausgeführt wird, gebildet, **dadurch gekennzeichnet, dass** das Pulver (302) antibakteriell wirksame Wirkbestandteile (104) umfasst und die thermische Energie eines mittels thermischen Spritzverfahren erzeugten Freistrahls während der Herstellung der Deckschicht (103) derart auf den Schmelzpunkt der Partikel des Pulvers (302) abgestimmt ist, dass die Deckschicht (103) aus vollständig aufgeschmolzenen und/oder nur teilweise aufgeschmolzenen und/oder noch unaufgeschmolzenen Wirkbestandteilen (104, 31, 32, 38; 34, 35, 36, 39) gebildet wird, mit oder ohne aufgeschmolzene und/oder teilgeschmolzene und/oder ungeschmolzenen Füllkörnern (33, 37).

10. Verfahren nach Anspruch 9, **dadurch gekennzeichnet, dass** die konkreten, der Kalziumphosphat-Spritzpulverkörnung angepassten Metallportionen erst während der Schichtherstellung über eine zweite, separate Pulverleitung in die Deckschicht (103) eingelagert werden, teilweise in Form von geschmolzenen Spritzlamellen (38) und/oder ungeschmolzen in der ursprünglichen Form (39) und/oder dass gleichzeitig die thermische Energie zur Erzeugung des Freistrahls den Anteil der kristallinen und/oder amorphen Schichtbestandteile und dadurch die Freisetzung der Wirkbestandteile regelt.

11. Verfahren nach einem der vorangegangenen Ansprüche, **dadurch gekennzeichnet, dass** die Deckschicht (103) in Abhängigkeit ihrer Schichtdicke mit variierender Zusammensetzung von Wirkbestandteilen und Füllbestandteilen entsteht.

12. Durch thermisches Spritzen hergestellte antibakterielle und osseointegrierende Kalziumphosphatdeckschicht (103)für ein Implantat, **dadurch gekennzeichnet, dass** der Kalziumphosphatschichtanteil mit eingelagerten metallenthaltenden Wirkbestandteilen ein eingestelltes Verhältnis kristalliner zu amorpher Schichtstruktur besitzt, wobei sich das Verhältnis in Abhängigkeit der Dicke der Deckschicht (103) verändert und so über eine gradierte Löslichkeit besitzt.

13. Deckschicht nach Anspruch 12, **dadurch gekennzeichnet, dass** ihre gradierte Löslichkeit in Richtung Schichtoberfläche höher und in unteren Schichtlagen niedriger ist oder dass ihre gradierte Löslichkeit in Richtung Schichtoberfläche niedriger und in unteren Schichtlagen höher ist.

14. Deckschicht nach Anspruch 12 oder 13, **dadurch gekennzeichnet, dass** eine zusätzliche Kalziumphosphatspritzschicht (44) ohne Wirkbestandteile (104) und mit hoher Löslichkeit als TCP-Schicht oder hochamorpher HA-Schicht oder als TCP/HA-Mischschicht ausgebildet ist.

15. Deckschicht (103) nach einem der vorhergehenden Ansprüche, **dadurch gekennzeichnet, dass** die Wirkbestandteile (104) im Spritzpulver so festgelegt sind, dass die mittels thermischer Spritztechnik daraus hergestellte Deckschicht (103) während der vorgesehenen Verweilzeit im Knochengewebe einerseits genügend gut antibakteriell wirkt, auf der anderen Seite nur so viel Metallionen und/oder Metallatome freisetzt, dass sie definitiv nicht toxisch wirkt.

## Claims

1. Prosthesis implantable without cement for traumatology and/or orthopaedics (40, 50, 100, 209, 310) made of biocompatible materials, which has a main body (100) with an anchoring region (101) which anchors in bone or tissue, the anchoring region (101) being provided at least partially with a covering layer (103), the covering layer (103) being formed from a powder (302) using a thermal spraying method preferably by a plasma spraying method, **characterized in that** the powder (302) consists essentially of calcium phosphate and comprises antibacterially effective active constituents (104) and preferably the covering layer (103) of the anchoring region (101) provided for contact with bone or tissue is applied either directly on the main body (100) or with inclusion of at least one laminated interlayer (Z) on the main body (100), in particular in a region (102).

2. Implant (40, 50, 100, 209, 310) according to the preceding claim, **characterized in that** the antibacterially effective active constituents (104) of the covering layer (103) consist of metal and/or precious metal, preferably of silver (12, 13) and/or comprise copper and/or zinc and mixtures thereof, wherein the active constituents (104) can be in atomic and/or ionic form and/or integrated in the crystal lattice and/or as concrete portions of material and the release of the antibacterially effective metal ions can be controlled via the solubility of the calcium phosphate.

3. Implant (40, 50, 100, 209, 310) according to one of the preceding claims, **characterized in that** the active constituents (104) in concrete portions of material are incorporated in the covering layer (103) preferably either unmelted as particles (12, 22b) or as spray powder grains (34, 35, 36, 39) with a diameter from 0.5 to preferably 25 micrometers and/or as melted sprayed lamellae (22a, 31, 32, 38).

4. Implant (40, 50, 100, 209, 310) according to one of the preceding claims, **characterized in that** the powder (302) for the production of the covering layer (103) consists essentially of calcium phosphate, in particular of hydroxyapatite and contains a defined proportion of active constituent (104) of metal and/or precious metal, preferably silver and/or copper and/or zinc and/or mixtures thereof, the metal content being in atomic and/or ionic form and/or mixed in as a concrete portion of material.

5. Implant (40, 50, 100, 209, 310) according to one of the preceding claims, **characterized in that** in the powder (302), a defined number of Ca ions in the crystal lattice of the calcium phosphate are replaced by ion exchange with metal ions, in particular Ag ions and/or metal atoms are incorporated at interstitial sites.

6. Implant (40, 50, 100, 209, 310) according to one of the preceding claims, **characterized in that** the ion exchange and/or the incorporation of metals take place during precipitation of the desired calcium phosphate from aqueous solution by adding metal salts in an established ratio to the components required for the precipitation of calcium phosphate, with the number of metal ions incorporated and/or metal atoms intercalated being determined taking into account specified reaction times and temperatures.

7. Implant (40, 50, 100, 209, 310) according to one of the preceding claims, **characterized in that** ion exchange and/or the intercalation of metal atoms is carried out on the prepared calcium phosphate spray powder grains, which are in the optimized distribution for the selected thermal spraying method for production of the covering layer (103).

8. Implant (40, 50, 100, 209, 310) according to one of the preceding claims, **characterized in that** the calcium phosphate spray powder consisting of filler grains (37) and/or active grains (34, 35, 36) is mixed with metal grains, which are adjusted to the grain size distribution of the calcium phosphate powder and preferably are formed not smaller than 5 µm and not larger than 25 µm.

9. Method for coating a prosthesis that is implantable without cement for traumatology and/or orthopaedics (40, 50, 100, 209, 310) or a part thereof, with an antibacterially effective covering layer (103), formed from a powder (302) using a thermal spraying method, which is preferably carried out as a plasma spraying method, **characterized in that** the powder (302) comprises antibacterially effective active constituents (104) and the thermal energy of a free jet produced by thermal spraying methods is adjusted during production of the covering layer (103) to the melting point of the particles of the powder (302) in such a way that the covering layer (103) is formed from completely melted and/or only partially melted and/or still unmelted active constituents (104, 31, 32, 38; 34, 35, 36, 39), with or without melted and/or partially melted and/or unmelted filler grains (33, 37).

10. Method according to Claim 9, **characterized in that** the concrete portions of metal matching the calcium phosphate spray powder granulometry are only incorporated in the covering layer (103) during layer production via a second, separate powder line, partially in the form of melted sprayed lamellae (38) and/or unmelted in the original form (39) and/or **in that** the thermal energy for production of the free jet simultaneously controls the proportion of crystalline and/or amorphous constituents of the layer and hence the release of the active constituents.

11. Method according to one of the preceding claims, **characterized in that** the covering layer (103) is formed, depending on its layer thickness, with varying composition of active constituents and filler constituents.

12. Antibacterial and osseointegrating calcium phosphate covering layer (103), produced by thermal spraying, for an implant, **characterized in that** the calcium phosphate fraction of the layer incorporating metal-containing active constituents has a set ratio of crystalline to amorphous layer structure, with the ratio varying depending on the thickness of the covering layer (103) and thus having graduated solubility.

13. Covering layer according to Claim 12, **characterized in that** its graduated solubility is higher in the direction towards the layer surface and is lower in the bottom layers **in that** its graduated solubility is lower in the direction towards the layer surface and is higher in the bottom layers.

14. Covering layer according to Claim 12 or 13, **characterized in that** an additional calcium phosphate sprayed layer (44) without active constituents (104) and with high solubility is formed as a TCP layer or highly amorphous HA layer or as TCP/HA mixed layer.

15. Covering layer (103) according to one of the preceding claims, **characterized in that** the active constituents (104) in the spray powder are established so that the covering layer (103) produced therefrom by thermal spraying technology on the one hand has sufficiently good antibacterial action, and on the other hand only releases an amount of metal ions and/or metal atoms that definitely do not have a toxic action, during the envisaged dwell time in the bone tissue.

## Revendications

1. Prothèse implantable sans ciment pour la traumatologie et/ou l'orthopédie (40, 50, 100, 209, 310) en matériaux biocompatibles, qui présente un corps de base (100) avec une zone d'ancrage (101) à ancrer dans un os ou un tissu, dans laquelle la zone d'ancrage (101) est munie au moins localement d'une couche de revêtement (103), dans laquelle la couche de revêtement (103) est formée d'une poudre (302) par utilisation d'un procédé de projection à chaud, de préférence un procédé de projection au plasma, **caractérisée en ce que** la poudre (302) se compose essentiellement de phosphate de calcium et comprend des composants actifs avec une action antibactérienne (104) et de préférence la couche de revêtement (103) de la zone d'ancrage (101) prévue pour un contact avec l'os ou le tissu est déposée soit directement sur le corps de base (100) soit avec interposition d'au moins une couche intermédiaire monocouche (Z) sur le corps de base (100), en particulier dans une région (102).

2. Implant (40, 50, 100, 209, 310) selon la revendication précédente, **caractérisé en ce que** les composants actifs à action antibactérienne (104) de la couche de revêtement (103) sont composés de métal et/ou de métal noble, de préférence d'argent (12, 13) et/ou de cuivre et/ou de zinc et comprennent des mélanges de ceux-ci, dans lequel les composants actifs (104) peuvent se présenter sous forme atomique et/ou ionique et/ou intégrée dans le réseau cristallin et/ou de portions de matière concrètes et la libération des ions métalliques à action antibactérienne peut être influencée par la solubilité du phosphate de calcium.

3. Implant (40, 50, 100, 209, 310) selon l'une quelconque des revendications précédentes, **caractérisé en ce que** les composants actifs (104) en portions de matière concrètes sont incorporés dans la couche de revêtement (103) de préférence soit à l'état non fondu sous forme de particules (12, 22b) ou de grains de poudre à projeter (34, 35, 36, 39) avec un diamètre de 0,5 à de préférence 25 micromètres et/ou sous forme de lamelles à projeter fondues (22a, 31, 32, 38).

4. Implant (40, 50, 100, 209, 310) selon l'une quelconque des revendications précédentes, **caractérisé en ce que** la poudre (302) pour la réalisation de la couche de revêtement (103) se compose essentiellement de phosphate de calcium, en particulier d'hydroxylapatite et contient une proportion définie de composant actif (104) en métal et/ou en métal noble, de préférence d'argent et/ou de cuivre et/ou de zinc et/ou de mélanges de ceux-ci, dans lequel la teneur en métal se présente sous forme atomique et/ou ionique et/ou est incorporée sous forme de portions de matière concrètes.

5. Implant (40, 50, 100, 209, 310) selon l'une quelconque des revendications précédentes, **caractérisé en ce qu'**un nombre fixe d'ions Ca sont remplacés dans le réseau cristallin du phosphate de calcium par des ions métalliques, en particulier des ions Ag, par un échange d'ions, et/ou des atomes métalliques sont insérés à des emplacements intermédiaires du réseau.

6. Implant (40, 50, 100, 209, 310) selon l'une quelconque des revendications précédentes, **caractérisé en ce que** l'échange d'ions et/ou l'insertion des métaux pendant la précipitation du phosphate de calcium désiré à partir d'une solution aqueuse est effectuée par ajout de sels métalliques dans une proportion fixée par rapport aux composants nécessaires pour la précipitation du phosphate de calcium, dans lequel le nombre des ions métalliques incorporés et/ou des atomes métalliques insérés est déterminé en tenant compte de durées et de températures de réaction prédéterminées.

7. Implant (40, 50, 100, 209, 310) selon l'une quelconque des revendications précédentes, **caractérisé en ce que** l'échange d'ions et/ou l'insertion des atomes métalliques est effectué(e) sur des grains de poudre à projeter de phosphate de calcium, qui sont présents dans la répartition optimisée pour le procédé de projection à chaud choisi pour la réalisation de la couche de revêtement (103).

8. Implant (40, 50, 100, 209, 310) selon l'une quelconque des revendications précédentes, **caractérisé en ce que** la poudre à projeter de phosphate de calcium se composant de grains de remplissage (37) et/ou de grains actifs (34, 35, 36) est mélangée avec des grains métalliques, qui sont adaptés à la répartition granulométrique de la poudre de phosphate de calcium et ne sont de préférence pas plus petits que 5 µm et pas plus gros que 25 µm.

9. Procédé de revêtement d'une prothèse implantable sans ciment pour la traumatologie et/ou l'orthopédie (40, 50, 100, 209, 310) ou d'une partie de celle-ci, avec une couche de revêtement active à action antibactérienne (103), en une poudre (302) formée par utilisation d'un procédé de projection à chaud, qui est exécuté de préférence sous forme de procédé de projection au plasma, **caractérisé en ce que** la poudre (302) comprend des composants actifs à action antibactérienne (104) et l'énergie thermique d'un jet libre produit au moyen du procédé de projection à chaud pendant la réalisation de la couche de revêtement (103) est adaptée au point de fusion des particules de la poudre (302), **en ce que** la couche de revêtement (103) est formée à partir de composants actifs complètement fondus et/ou seulement partiellement fondus et/ou encore non fondus (104, 31, 32, 38; 34, 35, 36, 39), avec ou sans grains de remplissage (33, 37) fondus et/ou partiellement fondus et/ou non fondus.

10. Procédé selon la revendication 9, **caractérisé en ce que** les portions de métal concrètes adaptées à la granulométrie de la poudre à projeter de phosphate de calcium ne sont insérées dans la couche de revêtement (103) que pendant la réalisation de la couche par une deuxième conduite à poudre séparée, en partie sous forme de lamelles à projeter fondues (38) et/ou à l'état non fondu dans leur forme initiale (39) et/ou **en ce qu'**en même temps l'énergie thermique pour la production du jet libre règle la proportion des composants cristallins et/ou amorphes de la couche et ainsi la libération des composants actifs.

11. Procédé selon l'une quelconque des revendications précédentes, **caractérisé en ce que** la couche de revêtement (103) apparaît en fonction de son épaisseur avec une composition variable de composants actifs et de composants de remplissage.

12. Couche de revêtement de phosphate de calcium antibactérienne et à intégration osseuse réalisée par projection à chaud (103) pour un implant, **caractérisée en ce que** la proportion de couche de phosphate de calcium avec des composants actifs insérés contenant des métaux possède une proportion réglée de structure de couche cristalline et amorphe, dans laquelle la proportion varie en fonction de l'épaisseur de la couche de revêtement (103) et présente ainsi une solubilité graduelle.

13. Couche de revêtement selon la revendication 12, **caractérisée en ce que** sa solubilité graduelle est plus élevée en direction de la surface de la couche et est plus faible dans les couches inférieures de la couche ou **en ce que** sa solubilité graduelle est plus faible en direction de la surface de la couche et est plus élevée dans les couches inférieures de la couche.

14. Couche de revêtement selon la revendication 12 ou 13, **caractérisée en ce qu'**une couche projetée de phosphate de calcium additionnelle (44) sans composants actifs (104) et avec une haute solubilité est formée par une couche TCP ou une couche hautement amorphe HA ou une couche mixte TCP/HA.

15. Couche de revêtement (103) selon l'une quelconque des revendications précédentes, **caractérisée en ce que** les composants actifs (104) dans la poudre à projeter sont fixés de telle manière que la couche de revêtement (103) réalisée à partir de ceux-ci par projection à chaud ait d'une part une action antibactérienne suffisamment bonne pendant la durée de séjour prévue dans le tissu osseux et ne libère d'autre part qu'une quantité d'ions métalliques et/ou d'atomes métalliques telle qu'elle ne soit définitivement pas toxique.
